# EUROPEAN PATENT SPECIFICATION

(11) **EP 2 976 067 B1**
(45) Date of publication and mention of the grant of the patent: **15.12.2021**
(21) Application number: 14715539.4
(22) Date of filing: 17.03.2014
(51) Int. Cl.: A61K 9/20, A61K 31/4709

(54) **PHARMACEUTICAL COMPOSITION COMPRISING A FLUOROQUINOLONE ANTIBACTERIAL AGENT AND METHOD FOR THE PREPARATION THEREOF**
PHARMAZEUTISCHE ZUSAMMENSETZUNG MIT EINEM ANTIBAKTERIELLEN FLUORCHINOLONWIRKSTOFF UND VERFAHREN ZUR HERSTELLUNG DAVON
COMPOSITION PHARMACEUTIQUE COMPRENANT UN AGENT ANTIBACTÉRIEN DE FLUOROQUINOLONE ET SON PROCÉDÉ DE PRÉPARATION

(30) Priority: 19.03.2013 GR 20130100162
(43) Date of publication of application: 27.01.2016
(73) Proprietor: Pharmathen S.A., 15351 Pallini Attikis (GR)
(72) Inventor: KARAVAS, Evangelos, GR-153 51 Pallini Attikis (GR); KOUTRIS, Efthimios, GR-153 51 Pallini Attikis (GR); SAMARA, Vasiliki, GR-153 51 Pallini Attikis (GR); KOUTRI, Ioanna, GR-153 51 Pallini Attikis (GR); ILIOPOULOU, Athina, GR-153 51 Pallini Attikis (GR); GOTZAMANIS, George, GR-153 51 Pallini Attikis (GR); ABATZIS, MORFIS, GR-153 51 Pallini Attikis (GR)
(86) International application number: PCT/EP2014/000712
(87) International publication number: WO 2014/146775

(56) References cited:
- EP-A1- 1 880 722
- WO-A1-03/101431
- WO-A1-2005/020998
- WO-A1-2013/190111
- CA-A1- 2 239 931
- US-A1- 2011 293 717
- MOHANACHANDRAN P S ET AL: "Superdisintegrants: An overview", INTERNATIONAL JOURNAL OF PHARMACEUTICAL SCIENCES REVIEW AND RESEARCH, GLOBAL RESEARCH ONLINE PUBLISHING HOUSE, IN, vol. 6, no. 1, 1 January 2011 (2011-01-01) , pages 105-109, XP002755784, ISSN: 0976-044X

## Description

### TECHNICAL FIELD OF THE INVENTION

The present invention relates to a stable pharmaceutical formulation for oral administration containing a therapeutically effective quantity of the fluoroquinolone antibacterial agent Moxifloxacin as crystalline Moxifloxacin hydrochloride anhydrous and a method for the preparation thereof.

### BACKGROUND OF THE INVENTION

Quinolones are synthetic antibacterial drugs, having been synthesized since 1960's. Even though originally used for the treatment of urinary tract infections, by the development of new core and side chain structures, have been evolved to wide spectrum antibacterial drugs. The launch of fluoroquinolones early 2000, was a major breakthrough and allowed treatment of a vast number of bacterial related diseases.

Fluoroquinolones are a family of synthetic broad-spectrum antibiotics, which eradicate bacteria by interfering with DNA replication. Moxifloxacin is one of the most commonly used fluoroquinolone antibiotic nowadays. Although the mechanism of action of quinolones in general is not fully known, Moxifloxacin acts through inhibition of the DNA gyrase activity. Also, inhibits topoisomerase IV, an enzyme structurally similar to DNA gyrase and essential for bacterial DNA replication.

Moxifloxacin is used to treat bacterial infections in patients of 18 years and older caused by bacteria susceptible to the drug. Moxifloxacin can be used for treatment of acute bacterial sinusitis (adequately diagnosed), acute exacerbations of chronic bronchitis (adequately diagnosed), community acquired pneumonia, mild to moderate pelvic inflammatory disease (i.e. infections of female upper genital tract, including salpingitis and endometritis), community-acquired pneumonia and complicated skin and skin structure infections.

The chemical name of Moxifloxacin is 1-cyclopropyl-7-[(1*S*, 6*S*)-2, 8-diazabicyclo [4.3.0] non-8-yl]-6-fluoro-8-methoxy-4-oxo- quinoline-3-carboxylic acid. The molecular formula is C₂₁H₂₄FN₃O₄ corresponding to a molecular weight of 401.431. It is a yellow crystalline powder, slightly hygroscopic, that may exist in several polymorphic forms. Moxifloxacin hydrochloride is sparingly soluble in water, slightly soluble in ethanol (96%) and practically insoluble in methanol. Moxifloxacin is public available in the pharmaceutical form of film coated tablets, eye drops and for intravenous administration.

EP 1 128 831 B1 discloses a film coated tablet formulation of Moxifloxacin prepared by wet granulation comprising a binder, a disintegrant, a lubricant and 2.5% to 25%wt lactose. According to the invention the use of lactose in the claimed range provides a composition of excellent hardness, friability and release properties.

Although the above mentioned patent represents an attempt to provide a film coated tablet of Moxifloxacin with adequate physical characteristics, there still exists a need for alternative formulations providing as well adequate chemical characteristics such as the stability of the pharmaceutical composition during storage time.

### SUMMARY OF THE INVENTION

It is, therefore, an object of the present invention to provide a stable solid dosage formulation for oral administration containing the fluoroquinolone antibiotic Moxifloxacin as crystalline Moxifloxacin hydrochloride anhydrous as an active ingredient, which exhibits excellent physicochemical properties providing a uniform and quick rate of release.

Another object of the present invention is to provide an improved stable solid dosage formulation for oral administration containing Moxifloxacin as crystalline Moxifloxacin hydrochloride anhydrous as an active ingredient, which overcomes the deficiencies of the prior art and avoids the degradation and polymorphic transformation of the active pharmaceutical ingredient resulting in longer shelf-life of the product.

A further approach of the present invention is to provide a method for the preparation of a stable solid dosage formulation for oral administration containing Moxifloxacin as crystalline Moxifloxacin hydrochloride anhydrous that overcomes the low flow properties of the active ingredient but yet can be manufacturing in quick and cost effective method.

In accordance with the above objects of the present invention, a pharmaceutical composition for oral administration is provided comprising Moxifloxacin as crystalline Moxifloxacin hydrochloride anhydrous as an active ingredient and an effective amount of the pharmaceutically acceptable water soluble diluent mannitol, a superdisintegrant added both intragranularly and extragranularly, and colloidal silicon dioxide added intragranularly in an amount of from 1%wt to 5%wt based on the total weight of the composition, in order to inhibit degradation and/or re-crystallization.

According to another embodiment of the present invention, a process for the preparation of a stable, solid dosage form for oral administration, containing Moxifloxacin as crystalline Moxifloxacin hydrochloride and an effective amount of the pharmaceutically acceptable water soluble diluent mannitol, a superdisintegrant added both intragranularly and extragranularly, and colloidal silicon dioxide added intragranularly in an amount of from 1%wt to 5%wt based on the total weight of the composition, in order to inhibit degradation and/or re-crystallization is provided, which comprises:
- Weighing of Moxifloxacin and all excipients and sieving.
- Mixing Moxifloxacin and the excipients of the internal phase until complete uniformity.
- Slugging or roller-compacting the above mixture.
- Sifting the slugs/flakes through a sieve.
- Mixing the sieved granules with the excipients of the external phase until complete homogeneity.
- Compressing the resulted mixture into a tablet dosage form.
- Optionally applying a film-coating on the core.

Other objects and advantages of the present invention will become apparent to those skilled in the art in view of the following detailed description.

### DETAILED DESCRIPTION OF THE INVENTION

For the purposes of the present invention, a pharmaceutical composition comprising an active ingredient is considered to be "stable" if said ingredient degrades less or more slowly than it does on its own and/or in known pharmaceutical compositions.

Moxifloxacin possesses several intrinsic physicochemical characteristics that cause certain problems during development of oral pharmaceutical compositions. In more detail, said drug substance has poor flow properties and low solubility in water and it has a tendency to transform into other polymorphic forms when formulated into a tablet.

It is well known that during the development of any pharmaceutical dosage form the impurities should be kept at very low limits, as said impurities might be toxic and harmful for the humans. Moreover, the impurities diminish the potency of pharmaceutical compositions during storage. They can also act as catalysts or intermediates in chemical reactions and change the drug into another form. Therefore, there is a need to develop a tablet that is stable and does not promote the degradation of the active pharmaceutical ingredient.

According to the present invention, a crystalline anhydrous form of Moxifloxacin hydrochloride was chosen to develop the tablet dosage form since in comparison to other forms, presented better flow properties. The use of such anhydrous form prohibits the use of wet granulation as a manufacturing process since such process would result in absorption of unwanted humidity. Furthermore, the use of direct compression was also not appropriate due to the poor flow properties of the active pharmaceutical ingredient. Dry granulation such as slugging or roller compactor was the process of choice.

It has been surprisingly found that the object of the present invention is achieved by the incorporation of specific excipients into the formulation, namely an effective amount of a pharmaceutically acceptable water soluble diluent such as mannitol, a disintegrant such as sodium starch glycolate and colloidal silicon dioxide.

According to the present invention the water soluble diluent mannitol is incorporated in the pharmaceutical composition. Mannitol is a non hygroscopic and no carcinogenic excipient used in pharmaceutical dosage forms for many years. Additionally, has very good flow properties and does not stick to the manufacturing equipment. Due to those properties the use of mannitol in amount of 10% to 15%wt was considered essential in the present invention.

Sodium starch glycolate is a super disintegrant the action of which relies on a combination of swelling and wicking of liquid into the tablet to generate rapid disintegration. It is freely flowing, has good storage stability and is not affected by high compression forces. The use at a range of 2% to 8%wt within the composition of the present invention provides quick and uniform dissolution of the active substance. The amount of the super disintegrant is splitted both intra and extra granularly for more adequate disintegration of the dosage form.

Colloidal silicon dioxide was also added in the composition according to the present invention. The addition of the excipient intragranularly improved the flow properties of the granule but most importantly the stability of the crystalline active substance form in its anhydrous state. The incorporation of colloidal silicon dioxide in an amount of 1% to 5%wt maintained Moxifloxacin in anhydrous form after stability studies. This is attributed to the fact that is a highly hygroscopic excipient that may absorb large quantities of humidity.

The pharmaceutical compositions of the present invention are characterized by excellent pharmacotechnical properties, such as flowability, compressibility and homogeneity. In more detail, the solid dosage forms of the present invention exhibit excellent pharmacotechnical characteristics including disintegration times, dissolution rates, hardness, friability, as well as chemical stability.

Moreover, the pharmaceutical compositions of the present invention may also contain one or more additional formulation excipient such as diluents, disintegrants, binders, lubricants, glidants and flavouring agents, provided that they are compatible with the active ingredient of the composition, so that it does not interfere with it in the composition and in order to increase the stability of the drug and the self-life of the pharmaceutical product.

Diluents may be, for example, microcrystalline cellulose, dextrates, dextrose, fructose, mannitol, sorbitol, starch, pregelatinized starch, sucrose, xylitol, maltose, maltodextrin, maltitol.

Disintegrants may be selected from alginic acid, carbon dioxide, carboxymethylcellulose calcium, carboxymethylcellulose sodium, croscarmellose sodium, guar gum, methylcellulose, polacrilin potassium, poloxamer, sodium alginate.

Binders may be, for example, alginic acid, carbomer, ethyl cellulose, gelatine, liquid glucose, guar gum, hydroxyethyl cellulose, methylcellulose, polydextrose, polyethylene oxide.

Also, at least a lubricant is incorporated into the formulation to prevent the powder from adhering to tablet punches during the compression procedure. Lubricants may be, for example, talc, magnesium stearate, calcium stearate, glyceryl behenate, hydrogenated castor oil, stearic acid, sodium lauryl sulphate.

Glidants are used to promote powder flow by reducing interparticle friction and cohesion. These are used in combination with lubricants as they have no ability to reduce die wall friction. Glidants may be, for example, talk, calcium silicate, calcium phosphate tribasic.

Finally, tablets of the present invention are optionally coated with a film composition that has no effect on the release of the active ingredient from the dosage form. The film-coating composition may comprise polymers, plasticizers, surfactants, opacifiers and/or pigments. The film-coating is prepared by dissolving the coating composition in water and/or organic solvent and then is sprayed on the tablet core.

Another embodiment of the present invention is the use of a cost effective process for the preparation of solid dosage forms containing Moxifloxacin or salts thereof.

Wet granulation techniques are avoided due to potential humidity absorption from the active substance that may occur when Moxifloxacin is incorporated in a pharmaceutical dosage form. Direct compression cannot be used due to pure flow properties of the active substance.

Thus, according to the present invention, dry granulation process was used for the preparation of solid dosage forms such as tablets containing Moxifloxacin or salt thereof, which is one of the most economical methods, as only standard equipment is used and the tablets produced by this method are also characterized by satisfactory physical resistance with no need for special packaging.

The main advantages of said process are that less equipment and space is required. Further, said process eliminates the need for binder solution, heavy mixing equipment and the costly and time consuming drying step required for wet granulation. Slugging has been proven particularly useful due to the fact that Moxifloxacin is moisture sensitive.

The following examples illustrate preferred embodiments in accordance with the present invention without limiting the scope of the invention.

### EXAMPLES

### Example 1

**Table 1: Compositions of Example 1**

| **Composition** | **1.1** | **1.2** | **1.3** |
|---|---|---|---|
| **Internal Phase** | **mg / tablet** | **mg / tablet** | **mg / tablet** |
| Moxifloxacin Base | **400,00** | **400,00** | **400,00** |
| Moxifloxacin HCl (anhydrous) | 436,37 | 436,37 | 436,37 |
| Microcrystalline Cellulose | 126,43 | 126,43 | 106,43 |
| Mannitol | 67,00 | 67,00 | 67,00 |
| Croscarmellose Sodium | - | 13,50 | - |
| Sodium Starch Glycolate | - | - | 13,50 |
| **External Phase** | | | |
| Croscarmellose Sodium | 33,50 | 20,00 | - |
| Sodium Starch Glycolate | - | - | 20,00 |
| Magnesium stearate | 6,70 | 6,70 | 6,70 |
| **Total for uncoated tablet** | **670,00** | **670,00** | **670,00** |

Three different formulations were prepared by dry granulation. The influence of intra and extragranular addition of a super disintegrant was observed. Mannitol was the diluents of choice for its excellent flow properties.

The manufacturing process for each tablet of formulations 1.1 to 1.3 was:
- The materials of the internal and external phase are weighed and sieved;
- The ingredients of the internal phase are mixed in a blender. Blending is performed until uniformity of the powder;
- Slugging of the mixture obtained from the previous step;
- Sizing of the slugs;
- Addition and mixing of the excipients of the external phase;
- Lubrication and compression of the granules into tablets.

The dissolution rate of the drug substance was studied in the three formulations by using a paddle apparatus at 50rpm and 0.1N HCl as dissolution medium. The results presented in table 2 below show a better and quicker profile when the super disintegrant was used both intra and extra granularly, as well as a better behaviour of sodium starch glycolate in comparison to croscarmellose sodium.

**Table 2: Dissolution profiles of compositions of example 1**

| Time (minutes) | Composition | | |
|---|---|---|---|
| | 1.1 | 1.2 | 1.3 |
| **5** | 46,27 | 53,60 | 64,36 |
| **10** | 73,85 | 82,92 | 83,19 |
| **15** | 84,94 | 89,73 | 90,56 |
| **30** | 91,79 | 94,30 | 97,78 |
| **45** | 93,72 | 95,81 | 100,32 |
| **60** | 95,16 | 97,26 | 101,02 |

However, the flow properties of the whole granule was not adequate for large scale production.

### Example 2

**Table 2: Composition of Example 2**

| **Internal Phase** | **mg** / **tablet** |
|---|---|
| Moxifloxacin Base | **400,00** |
| Moxifloxacin HCl (anhydrous) | 436,37 |
| Microcrystalline Cellulose | 114,50 |
| Mannitol | 113,03 |
| Sodium Starch Glycolate | 12,30 |
| Magnesium stearate | 8,20 |
| **External Phase** | |
| Sodium Starch Glycolate | 28,70 |
| Talk | 24,60 |
| Magnesium stearate | 12,30 |
| **Total for uncoated tablet** | **750,00** |

Tablets of example 2 were prepared with the same manufacturing process as in example 1. In order to improve the granule flow properties of composition 1.3, magnesium stearate was added both intra and extra granularly. Additionally, talk was added in order to avoid sticking of the granule on the punches. Furthermore, mannitol content was also increased as well as the total sodium starch glycolate content in order to maintain dissolution profile similar to that of example 1.3.

The dissolution profile obtained from intact tablets of composition 2 was similar to that of composition 1.3. However, there was a problem on tablet appearance since most of them revealed capping.

### Example 3

**Table 3: Composition of Example 3**

| **Internal Phase** | **mg / tablet** |
|---|---|
| Moxifloxacin Base | **400,00** |
| Moxifloxacin HCl (anhydrous) | 436,37 |
| Microcrystalline Cellulose | 82,50 |
| Mannitol | 113,03 |
| Hydroxypropyl Cellulose | 41,00 |
| Sodium Starch Glycolate | 12,30 |
| Magnesium stearate | 8,20 |
| **External Phase** | |
| Microcrystalline Cellulose | 41,00 |
| Sodium Starch Glycolate | 28,70 |
| Talk | 24,60 |
| Magnesium stearate | 12,30 |
| ***Total for uncoated tablet*** | ***800,00*** |

Tablets of composition of example 3 were prepared with the same manufacturing process as in the previous examples. In order to avoid capping of the tablets hydroxypropyl cellulose was added intragranularly as a binder and the amount of microcrystalline cellulose used was splitted in both phases internal and external to increase cohesiveness of the granule.

All the physical properties of the tablets such as hardness, disintegration, friability as well as dissolution profile were adequate. In order to investigate the chemical stability, tablets were introduced in a stability chamber under accelerated conditions (40°C). After the first month, tablets were subjected to an XRD analysis in order to see if the crystalline form used remained the same. The XRD showed a form changement from the anhydrous crystalline form used.

### Example 4

**Table 4: Composition of Example 4**

| **Internal Phase** | **mg** / **tablet** |
|---|---|
| Moxifloxacin Base | **400,000** |
| Moxifloxacin HCl (anhydrous) | **436,370** |
| Microcrystalline Cellulose | 73,800 |
| Mannitol | 113,030 |
| Colloidal Silicon Dioxide | 28,700 |
| Sodium Starch Glycolate | 12,300 |
| Hydroxypropyl Cellulose | 41,000 |
| Magnesium stearate | 8,200 |
| **External Phase** | |
| Microcrystalline Cellulose | 41,000 |
| Sodium Starch Glycolate | 28,700 |
| Talk | 24,600 |
| Magnesium stearate | 12,300 |
| **Total for uncoated tablet** | **820,000** |

Tablets of composition of example 4, where prepared by the following manufacturing process:
- The materials of the internal and external phase as well as crystalline Moxifloxacin hydrochloride anhydrous are weighed and sieved;
- The ingredients of the internal phase, microcrystalline cellulose, mannitol, colloidal silicon dioxide, sodium starch glycolate, hydroxypropyl cellulose and magnesium stearate are mixed in a blender. Blending is performed until uniformity of the powder;
- Slugging of the mixture obtained from the previous step;
- Sizing of the slugs;
- Addition and mixing of the excipients of the external phase, microcrystalline cellulose and sodium starch glycolate;
- Lubrication with magnesium stearate and talk followed by compression of the granules into tablets.
- Optionally, film-coating the prepared tablets.

All the physical properties of the tablets such as hardness, disintegration, friability as well as dissolution profile were adequate. In order to investigate the chemical stability, tablets were introduced in a stability chamber under normal, intermediate and accelerated conditions according to current ICH guidelines. The results obtained as in table 5 below, present a particularly stable pharmaceutical composition.

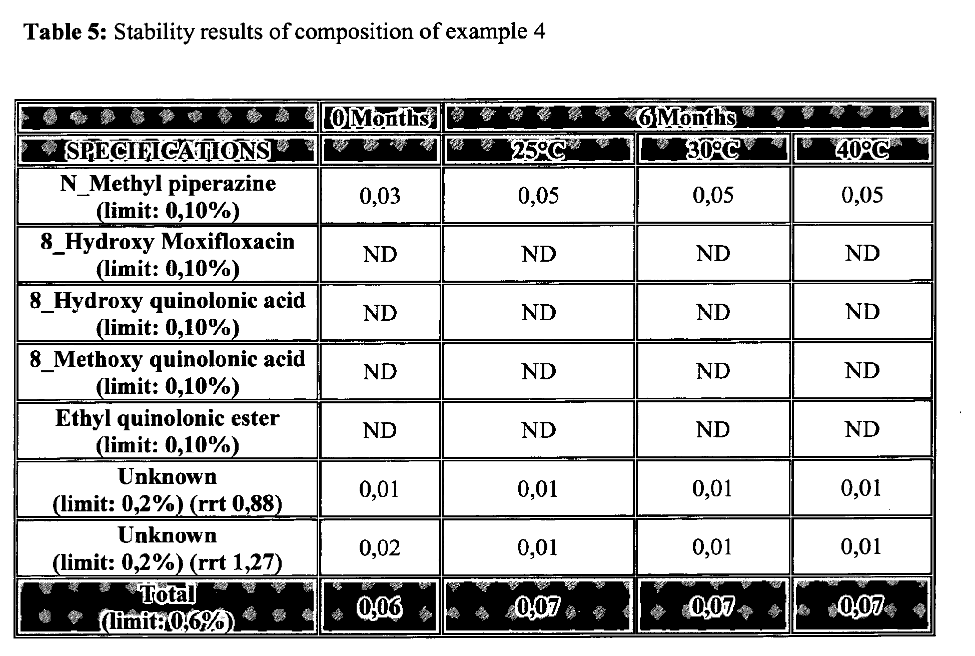

Additionally, tablets from normal conditions were subjected to an XRD analysis in order to see if the crystalline form used remained the same. The XRD analysis proved that the same crystalline anhydrous form was maintained.

## Claims

1. A pharmaceutical composition for oral administration comprising the fluoroquinolone antibiotic Moxifloxacin as crystalline Moxifloxacin hydrochloride anhydrous, mannitol, a superdisintegrant added both intragranularly and extragranularly and colloidal silicon dioxide added intragranularly in an amount of from 1%wt to 5%wt based on the total weight of the composition.

2. The pharmaceutical composition according to claim 1, wherein mannitol is added in an amount of from 10%wt to 15%wt based on the total weight of the composition.

3. The pharmaceutical composition according to claim 1, wherein the superdisintegrant is sodium starch glycolate and is added in an amount of from 2%wt to 8%wt based on the total weight of the composition.

4. The pharmaceutical composition according to any preceding claim, wherein the composition comprises at least one additional pharmaceutically inert excipient selected from diluents, binders, compression aids, disintegrants, glidants, lubricants, flavours, water scavengers, colorants and sweeteners.

5. A process for the preparation of a pharmaceutical composition for oral administration comprising the fluoroquinolone antibiotic Moxifloxacin as crystalline Moxifloxacin hydrochloride anhydrous, mannitol, a superdisintegrant added both intragranularly and extragranularly and colloidal silicon dioxide added intragranularly in an amount of from 1%wt to 5%wt based on the total weight of the composition:
- Weighing of the active ingredient, all excipients and sieving;
- Mixing the active ingredient, the total amount of colloidal silicon dioxide, the total amount of mannitol, a portion of the superdisintegrant and any additional inert excipients of the internal phase until complete uniformity;
- Slugging or roller-compacting the above mixture;
- Sifting the slugs/flakes through a sieve;
- Mixing the sieved granules with the remaining portion of the superdisintegrant and any additional excipient of the external phase until complete homogeneity;
- Lubricating the mixture;
- Compressing the resulted mixture into a tablet dosage form;
- Optionally applying a film-coating on the core.

## Patentansprüche

1. Pharmazeutische Zusammensetzung zur oralen Darreichung bestehend aus dem Antibiotikum Moxifloxacin, welches der Fluorchinolone zugerechnet wird, in der kristallinen wasserfreien Form von Moxifloxacin als Hydrochlorid, sowie aus Mannitol, einem Supersprengmittel, das sowohl intragranular als auch extragranular hinzugefügt wird, einem kolloidalen Siliciumdioxid, das intragranular in einem Gewichtprozent zwischen 1% und 5% des gesammten Gewichts der Zusammensetzung hinzugefügt wird.

2. Pharmazeutische Zusammensetzung nach Anspruch 1, **dadurch gekennzeichnet, dass** Mannitol in einem Gewichtsprozent zwischen 10% und 15% des gesammten Gewichts der Zusammensetzung hinzugefügt wird.

3. Pharmazeutische Zusammensetzung nach Anspruch 1, **dadurch gekennzeichnet, dass** das Supersprengmittel in einem Gewichtsprozent zwischen 2% und 8% des gesammten Gewichts der Zusammensetzung hinzugefügt wird.

4. Pharmazeutische Zusammensetzung nach einem der vorhergehenden Ansprüche, **dadurch gekennzeichnet, dass** die Zusammensetzung mindestens noch einen pharmazeutisch inaktiven Hilfsstoff umfasst, z.B. Zerfallsmittel, Bindemittel, Kompressionshilfsstoffe, Streckmittel, Gleitmittel, Schmiermittel, Aromastoffe, Wasserfänger, Farbstoffe und Süßstoffe.

5. Verfahren für die Zubereitung einer pharmazeutischen Zusammensetzung zur oralen Darreichung bestehend aus dem Antibiotikum Moxifloxacin, welches der Fluorchinolone zugerechnet wird, in der kristallinen wasserfreien Form von Moxifloxacin als Hydrochlorid, sowie aus Mannitol, einem Supersprengmittel, das sowohl intragranular als auch extragranular hinzugefügt wird, einem kolloidalen Siliciumdioxid, das intragranular in einem Gewichtprozent zwischen 1% und 5% des gesammten Gewichts der Zusammensetzung hinzugefügt wird.
- Den Wirkstoff und sämtliche Hilfsstoffe wiegen und sieben;
- Den Wirkstoff, die gesamte Menge des kolloidalen Siliciumdioxids, die gesamte Menge von Mannitol, eine Portion des Supersprengmittels und ggf. die zusätzlichen inaktiven Hilfsstoffe der internen Phase so lange vermischen, bis die Mischung vollständig einheitlich ist;
- Die o.g. Mischung mittels Slugging oder Walzenkompaktierung pressen;
- Die Slugs/Flocken sieben;
- Das Granulat nach dem Sieben mit der übriggebliebenen Portion des Supersprengmittels und den ggf. weiteren Hilfsstoffen der externen Phase vermischen, bis die Mischung komplett einheitlich wird;
- Die Mischung schmieren;
- Die daraus resultierende Mischung tablettieren;
- Optional die Tabletten mit Film überziehen.

## Revendications

1. Composition pharmaceutique pour administration orale comprenant l'antibiotique fluoroquinolone moxifloxacine sous forme de chlorhydrate de moxifloxacine cristallin anhydre, du mannitol, un superdésintégrant ajouté à la fois par voie intragranulaire et extragranulaire et du dioxyde de silicium colloïdal ajouté par voie intragranulaire en une quantité de 1 % à 5 % (p/p) sur la base du poids total de la composition.

2. Composition pharmaceutique selon la 1ère revendication, dans laquelle le mannitol est ajouté en une quantité de 10 % à 15 % (p/p) sur la base du poids total de la composition.

3. Composition pharmaceutique selon la 1ère revendication, dans laquelle le superdésintégrant est le glycolate d'amidon sodique et est ajouté en une quantité de 2 % à 8 % (p/p) sur la base du poids total de la composition.

4. Composition pharmaceutique selon l'une quelconque des revendications précédentes, **caractérisée en ce qu'**elle comprend au moins un excipient additionnel pharmaceutiquement inerte choisi parmi les diluants, liants, aides à la compression, délitants, glissants, lubrifiants, arômes, fixateurs d'eau, colorants et édulcorants.

5. Composition pharmaceutique pour administration orale comprenant l'antibiotique fluoroquinolone moxifloxacine sous forme de chlorhydrate de moxifloxacine cristallin anhydre, du mannitol, un superdésintégrant ajouté à la fois par voie intragranulaire et extragranulaire et du dioxyde de silicium colloïdal ajouté par voie intragranulaire en une quantité de 1 % à 5 % (p/p) sur la base du poids total de la composition.
- Pesage du principe actif, de tous les excipients et tamisage ;
- Mélange du principe actif, de la quantité totale de dioxyde de silicium colloïdal, la quantité totale de mannitol, une partie du superdésintégrant et les éventuels excipients inertes supplémentaires de la phase interne jusqu'à complète homogénéité ;
- compression ou compactage au rouleau du mélange ci-dessus ;
- Tamisage des limaces/flocons à travers un tamis ;
- Mélange des granulés tamisés avec la partie restante du superdésintégrant et tout excipient supplémentaire de la phase externe jusqu'à complète homogénéité ;
- Lubrification du mélange ;
- Compression du mélange obtenu en une forme posologique de comprimés ;
- Application éventuelle d'un pelliculage sur le noyau.
